# EUROPEAN PATENT APPLICATION

(11) **EP 1 731 159 A1**
(43) Date of publication of application: **13.12.2006**
(21) Application number: 05721315.9
(22) Date of filing: 23.03.2005
(51) Int. Cl.: A61K 35/76, A01K 61/00, A23K 1/165, A23K 1/18, A61P 25/00, A61P 31/12

(54) **METHOD OF PREVENTING NODAVIRUS INFECTION AND THERAPEUTIC METHOD**

(30) Priority: 24.03.2004 JP 2004086929
(71) Applicant: Japan Science and Technology Agency, Kawaguchi-shi, Saitama 332-0012 (JP)
(72) Inventor: Nakai, Toshihiro, Higashihiroshima-shi, Hiroshima 7390023 (JP)
(74) Representative: TBK-Patent
(86) International application number: PCT/JP2005/005265
(87) International publication number: WO 2005/089781

(57) **Abstract**

The present invention provides a method for the prevention or the treatment of an infection of fishes with nodaviruses or a disease such as, for example, viral nervous necrosis or the like, caused by an infection with nodaviruses. The infection of fishes with nodavirus or the outbreak of a disease including viral nervous necrosis or the like caused by the infection with nodaviruses can be prevented or treated by transdermally administering fishes with aquabirnaviruses, for example, by injection, or dipping fishes in a solution containing an aquabirnavirus or supplying fishes with a feed containing an aquabirnavirus.

## Description

### Technical Field

The present invention relates to a method for the prevention of an infection with nodavirus and to a method for the prevention or the treatment of a disease caused by an infection of nodavirus. More particularly, the present invention relates to a method for the prevention of diseases, including particularly viral nervous necrosis and so on, caused by infections with nodaviruses and to a method for the treatment of such diseases, by using a non-pathogenic aquabirnavirus.

### Background Technology

At the present time, approximately forty species of marine fishes are cultivated in Japan in order to protect and ensure resources of marine fishes. Several species of juvenile fishes are also being produced as seedlings for raising or culturing fishes. Techniques for the production of seedlings of juvenile fishes and the technology for raising or culturing fishes have been developed, however, control of diseases caused by infections, particularly by infections with viruses, may so far have often been encountered with difficulties. For instance, it has been reported on VNN (viral nervous necrosis) and so on, which is to be caused by infections of yellowtails and so on with birnaviruses such as YAV (yellowtail ascites virus) or by infections of halibuts, flounders, groupers or the like with nodaviruses (Refer to, for example, Non-patent literature 1 and Non-patent literature 2).

Among those diseases, especially viral nervous necrosis caused by infections with nodaviruses has broken out in marine fishes throughout the world since the nineteen-nineties. This disease becomes a great threat to the seedling-producing facilities and the fish-raising industry because its lethality is found to be extremely high. It is further reported that over thirty species of fishes are infected (Refer to, for example, Non-patent literature 2).

Heretofore, it has also been reported that, although experiments have revealed that the vaccine therapy was effective for the viral nervous necrosis, it cannot be applied to juvenile fishes and newly hatched fishes because the vaccine therapy has to be carried out by injection. Further, the vaccine therapy using a published genetically engineered coat protein vaccine has the disadvantage that it requires a long duration of time as long as three weeks until it can demonstrate its activity (Refer to, for example, Non-patent literature 3 and Non-patent literature 4).

Fish nodaviruses may be classified into three different serum types and a vaccine has to be produced individually for each of the three different serum types (Refer to, for example, Non-patent literature 5).

Further, in order to prevent a vertical infection from parent fishes, virus-free parent fishes have to be selected, however, the technology for detection of viral genes, special apparatus for use therewith, and disinfection of fertilized eggs are required for the selection of parent fishes. On the other hand, apparatuses for use in disinfection with ozone or ultraviolet rays for sterilization of water for raising or culturing fishes are needed in order to prevent a horizontal infection. These preventive measures, however, have a large amount of labor and a huge amount of expenses (Refer to, for example, Non-patent literature 6 and Non-patent literature 7).

Moreover, it is of the present status that the effective method for the prevention and the treatment of the viral nervous necrosis, particularly for the prevention of an outbreak of the disease in the juvenile fish stage and thereafter, has not yet been put to practical use. Therefore, a strong demand has long been made to develop an effective method for the prevention and/or the treatment of the viral nervous necrosis for fishes in the juvenile fish stage and thereafter.

On the other hand, it has been reported that flounders having a primary infection with non-lethal aquabirnaviruses (ABV) has an increased resistance to a secondary infection with viral haemorrhagic septicaemia virus (VHSV) that is targeting various internal organs including, for example, the kidney and so on (Refer to, for example, Non-patent literature 8 and Non-patent literature 9). There has so far been no report, however, that the ABV is also effective for the viral nervous necrosis that is a disease that causes damages of the central nervous system by the infection with nodaviruses because the VHSV is the virus that targets the internal organs such as the kidney.

With the above-mentioned prior art technology taken into account as the background, the present inventors have found as a result of extensive researches on non-pathogenic infections with aquabirnaviruses (ABV) that the ABVs are effective for the viral nervous necrosis that is a disease caused by infections by nodaviruses. The present invention has been completed on the basis of this finding.

Therefore, the present invention has the object to provide a method for the prevention or the treatment of an infection with nodaviruses through an infection with a non-pathogenic aquabirnavirus.
Non-patent literature 1: Takano, R., et al., Fish Pathology, 36(3), 153-169, 2001.9
Non-patent literature 2: Munday, B. L., et al., Journal of Fish Diseases, 25, 127-142, 2002
Non-patent literature 3: Tanaka, S., et al., Journal of Fish Diseases, 24, 15-22, 2001;
Non-patent literature 4: Yuasa, K., et al., Journal of Fish Diseases, 25, 53-56, 2002
Non-patent literature 5: Mori, K., et al., Diseases of Aquatic Organisms, 57, 19-26, 2003
Non-patent literature 6: YOSHOKU, vol. 10, pp. 21-22, 2001
Non-patent literature 7: Tsuchihashi, Y., et al., SUISANZOSHOKU, 50(3), 355-361, 2002
Non-patent literature 8: Pakingking, R. Jr., et al., Fish & Shellfish Immunology, in the process of printing (2004);
Non-patent literature 9: Pakingking, R. Jr., et al., Fish Pathology, 38, 15-21, 2003

### Disclosure of the Invention

In order to achieve the above object, the present invention provides a method for the prevention of a disease to be caused by an infection with nodaviruses through an infection of a non-pathogenic aquabirnavirus.

The present invention also provides a method for the treatment of a disease to be caused by an infection with nodaviruses through a non-apparent infection with a non-pathogenic aquabirnavirus.

Further, the present invention provides a method for the prevention of the outbreak of a disease caused by the infection with nodaviruses or a method for the treatment of such a disease by orally administering an aquabirnavirus.

### Brief Description of the Accompanying Drawings

Figure 1 is a graph showing a variation of nodavirus potential values in the kidney and the brain of a bastard halibut.
Figure 2 is a graph showing an accumulated death ratio of a convict grouper by an infection with nodaviruses.
Figure 3 is a graph showing a variation of nodavirus potential values in the kidney and the brain of a bastard halibut.

### Best modes for carrying out the Invention

In accordance with the present invention, the method for the prevention of an infection with nodaviruses and the method for the treatment of diseases resulting from infections therewith are characterized in that the infection with nodaviruses can be prevented and/or a disease caused by the infection of nodaviruses can be treated by the application of aquabirnavirus (ABV) to fish species as a target, which can cause a non-apparent and non-pathogenic infection.

Although aquabirnaviruses (ABVs) are non-pathogenic or non-poisonous and non-hazardous to the environment, it is known that a number of natural fishes are subjected to a non-apparent infection with the ABVs.

The aquabirnaviruses to be used for the present invention can be isolated readily from the nature such as, for example, marine fishes subjected to a non-apparent infection, including but being not limited to, flounders, groupers, halibuts and so on. The non-poisonous or less poisonous ABVs isolated from the nature can be amplified readily by inoculation into cells of other fishes as well as host fishes and incubation of the cells thereof.

The application of aquabirnaviruses (ABVs) to fishes, which has been multiplied by incubation in the manner as described above, may be made, for example, by administration through injection of an ABV solution as well as by supplying a feed admixed with the ABVs or dipping a solution containing the ABVs. These application method are the secure methods that allow an infection of many natural fishes with the ABVs because in many cases they are orally infected by feeding plankton holding the ABVs.

In accordance with the present invention, in cases where a feed mixed with aquabirnaviruses (ABVs) is fed, for example, the ABV may be mixed generally at the rate of approximately 10⁷ to 10⁸ TCID₅₀ (Tissue Culture Infectious Dose 50%) per gram of the total feed. In cases where fishes are dipped in an ABV solution, for example, the ABV may be mixed generally at the rate of approximately 10⁵ to 10⁶ TCID₅₀ per milliliter of the solution. In cases where an ABV injection is used, for example, the ABV may be mixed generally at the rate of approximately 10⁷ to 10⁸ TCID₅₀ per millimeter of the injection.

The species of fishes to which the prevention and treatment method according to the present invention can be applied is not limited to particular ones and may include any fish that is infected with nodaviruses or is capable of being infected therewith. Moreover, it may range from seedlings to upbringing fishes including juvenile and parent fishes, regardless of whatever a size (a growth stage) it may be.

The present invention will be described in more details hereinafter by way of working examples. The working examples, however, will be interpreted in no respects to restrict the present invention in any way and described solely with the object to explain the present invention in a specific and illustrative manner.

### Example 1: Properties of Aquabirnavirus (an FBV strain)

An aquabirnavirus (an FBV strain) to be used for this working example belongs to the genus *Aquabirnavirus* of the family *Birnaviridae* and is a non-enveloped spherical virus having a diameter of approximately 60 nm. Further, the aquabirnavirus has a two-stranded RNA as a nucleic acid. The FBV strain also belongs to the genus *Aquabirnavirus* of the family *Birnaviridae* and it can be neutralized thoroughly by rabbit anti-serum prepared by YTAV (yellowtail ascites virus) which is known as a pathogenic virus for yellowtails. It is also classified as the serum type identical to that of YTAV. It is to be noted herein, however, that the FBV strain is found serologically different from IPNV (infectious pancreatic necrosis virus) which is known as a pathogenic salmon virus belonging to the family Birnaviridae. Further, the FBV strain was multiplied well with RTG-2 cells of an established cell line derived from a fish.

### Example 2: Test of Safety of Aquabirnavirus (an FBV strain) upon Bastard halibut (Paralichthys olivaceus)

An aquabirnavirus (an FBV strain) was obtained by incubation of RTG-2 cells in a culture medium (Eagle's MEM; fetal bovine serum). The FBV strain obtained in the manner as described above was then stored at the temperature of -80°C. This freeze-dried strain was found effective even after storage for six months and longer.

The FBV strain stored by freeze-drying in the manner as described above was then inoculated (at the rate of 10^{6.9} TCID₅₀ per fish; number of fishes tested: 20) into the muscle of Bastard halibuts (*P. olivaceus*) (artificially produced) each having an average weight of 18 grams. As a result of observations for three weeks at a water temperature of 20°C, it was found that neither dead fish nor abnormal fish were recognized. Further, no pathogenicity was recognized for the FBV strain with respect to young Bastard halibuts (having an average weight of 0.5 grams).

### Example 3: Test of Safety of Aquabirnavirus (an FBV strain) upon Convict grouper (Epinephelus septemfasciatus)

An aquabirnavirus (an FBV strain) was obtained by incubation of RTG-2 cells in a culture medium (Eagle's MEM; fetal bovine serum) in substantially the same manner as in Example 2. The FBV strain obtained in the manner as described above was stored in the state of being frozen at the temperature of -80°C. This freeze-dried strain was found effective even after storage for six months and longer.

The FBV strain stored by freeze-drying in the manner as described above was then inoculated (at the rate of 10^{7.1} TCID₅₀ per fish; number of fishes tested: 20) into the muscle of Convict groupers (*E. septemfasciatus)* (artificially produced) each having an average weight of 12 grams. As a result of observations for three weeks at a water temperature of 25°C, it was found that neither dead fish nor abnormal fish were recognized.

### Example 4: Effect of Aquabirnavirus (FBV strain) upon Inhibition of Infection of Bastard halibut with Nodavirus

An aquabirnavirus (an FBV strain) was obtained by incubation of RTG-2 cells in a culture medium (Eagle's MEM; fetal bovine serum) in substantially the same manner as in Example 2. The FBV strain obtained in the manner as described above was stored in the state of being frozen at the temperature of -80°C. This freeze-dried strain was found effective even after storage for six months and longer.

The FBV strain stored by freeze-drying in the manner as described above was then inoculated (at the inoculation rate of 10^{6.6} TCID₅₀ per fish) into the muscle of Bastard halibuts (artificially produced) each having an average weight of 12 grams and bred for seven days at the water temperature of 20°C. Thereafter, fish nodavirus RGNNV (an SGWak97 strain) incubated in E-11 cells was inoculated into the muscle of Bastard halibuts (at the inoculation rate of 10^{8.6} TCID₅₀ per fish) and measured at days 3, 7 and 14, respectively, for the amount of RGNNV in the kidney and the brain of the bastard halibuts.

As a control group, there were used fishes that were not inoculated with the FBV strain and were infected solely with the fish nodavirus RGNNV. In this example, it was further recognized that no fish was caused to be died due to the infection with the RGNNV regardless of whether it was inoculated with the FBV strain or not.

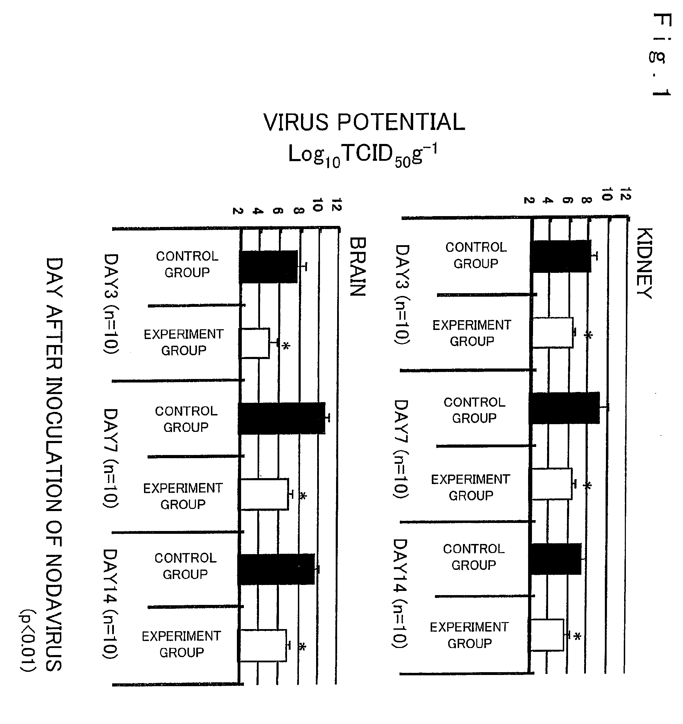

As shown in Figure 1, it was found that the amounts of RGNNV in the kidney and the brain of the Bastard halibut inoculated in advance with ABV were significantly lower than those of the control group at either of measurement times.

### Example 5: Effect of Aquabirnavirus (FBV strain) upon Inhibition of Infection of Convict grouper with Nodavirus

An aquabirnavirus (an FBV strain) was obtained by incubation of RTG-2 cells in a culture medium (Eagle's MEM; fetal bovine serum) in substantially the same manner as in Example 2. The FBV strain obtained in the manner as described above was stored in the state of being frozen in the manner as described above. The FBV strain stored by freeze-drying in the manner as described above was then inoculated (at the inoculation rate of 10^{7.1} TCID₅₀ per fish) into the muscle of Convict groupers (artificially produced) each having an average weight of 12 grams and bred for seven days at the water temperature of 25°C. Thereafter, fish nodavirus RGNNV (SGWak97 strain) incubated in E-11 cells was then inoculated into the muscle of the Convict groupers (at the inoculation rate of 10^{5.1} TCID₅₀ per fish). As a control group, there were used Convict groupers without inoculation of the FBV strain and with a sole infection with RGNNV.

In this example, a mortality of the fishes due to nodavirus infection was measured by observations for two weeks after the inoculation with the FBV strain and the amounts of the RGNNV in the kidney and the brain of the Convict groupers were measured at days 3, 7 and 14, respectively.

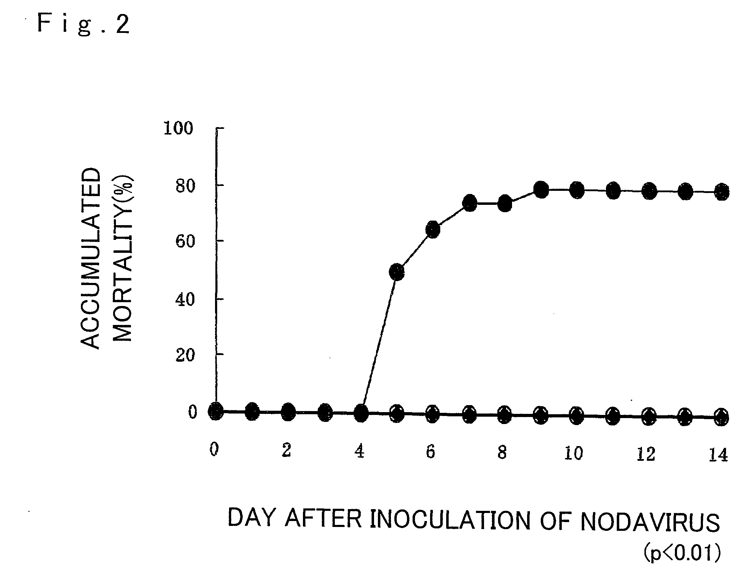

As a result, it was observed as shown in Figure 2 that the mortality of the fishes in the control group where no FBV strain was inoculated was 80%, while the mortality of the fishes with the FBV inoculated in advance was 0%.

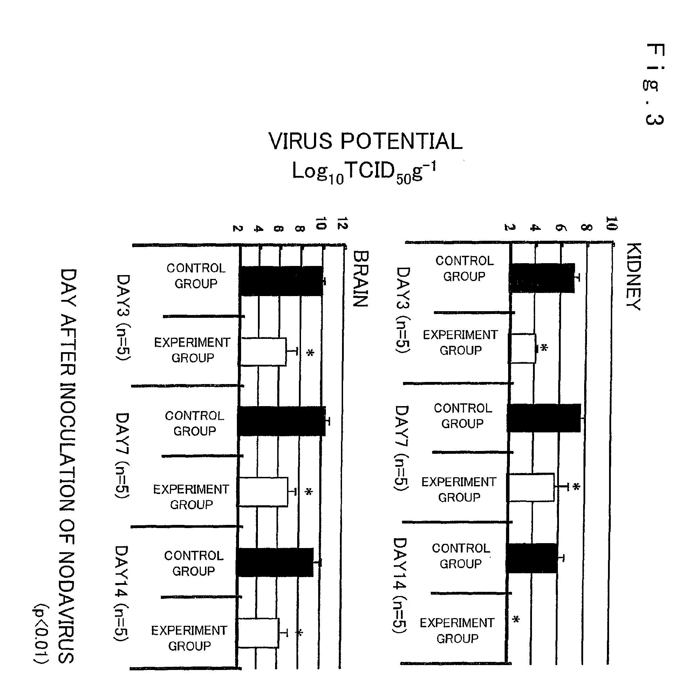

Further, as shown in Figure 3, it was found that the amounts of the RGNNV in the kidney and the brain of the fish inoculated in advance with the ABV were significantly lower than those of the control group at either of measurement times. Although not shown in this description as data, it was also observed that the RGNNV disappeared from the brain of the fish inoculated with the FBV strain.

### Industrial Applicability

The method for the prevention and the method for the treatment according to the present invention are found effective for a wide variety of fish species which are infected with viral nervous necrosis caused by infections with nodaviruses as well as with other infectious diseases caused by the infections with nodaviruses.

The method for the prevention and the method for the treatment according to the present invention can also present the effects that they can be applied to newly hatched and young fishes to which conventional treatment with vaccine cannot so far be applied successfully, because not only the administration can be conducted through oral routes but also the administration by means of the dipping method can be conducted in order to permit fishes to be orally administered through feeding.

Further, the anti-viral action of the aquabirnaviruses (ABVs) to be used for the present invention is non-specific so that it can be used regardless of a serum type. Therefore, the treatment method according to the present invention has the merits that, unlike conventional vaccine treatment, it does not require the production of vaccine for each of three different serum types of nodaviruses.

Moreover, the present invention has the great advantages that the inoculation of the aquabirnaviruses (ABVs) is expected to demonstrate the preventive effects for infections with nodaviruses and the treatment effects for parent fishes infected with nodaviruses in a non-apparent way.

Furthermore, the methods for the prevention and the treatment in accordance with the present invention have the great advantages that the administration of a combination of the aquabirnaviruses (ABVs) with vaccine is considered to induce not only a non-specific control but also a specific control.

In addition to the above advantages, the present invention is further provided with the advantages that, as no special apparatuses and so on are required for the methods for the prevention and the treatment in accordance with the present invention, expenses required for the preparation and administration of the ABV to be used for the present invention can be decreased to a great extent, as compared with conventional vaccine treatment.

The prevention method and the treatment method according to the present invention can also be applied to the production as well as incubation of seedlings of a wide variety of fish species.

## Claims

1. A method **characterized in that** an infection with a nodavirus or an outbreak of a disease caused by an infection with a nodavirus or said disease is prevented or treated by administering fish with an aquabirnavirus.

2. The method as claimed in claim 1, **characterized in that** said disease is viral nervous necrosis.

3. The method as claimed in claim 1 or 2, **characterized in that** said aquabirnavirus is administered by injection or through an oral route.

4. The method as claimed in any one of claims 1 to 3, **characterized in that** said aquabirnavirus is administered orally by dipping the fish in a solution containing said aquabirnavirus or the fish is fed with a feed with said aquabirnavirus mixed therewith.

5. The method as claimed in any one of claims 1 to 3, **characterized in that** said aquabirnavirus is administered by injection containing said aquabirnavirus at a rate of 10⁷ TCID₅₀ to 10⁸ TCID₅₀ per millimeter of the injection, by supplying a feed containing said aquabirnavirus at a rate of 10⁷ TCID₅₀ to 10⁸ TCID₅₀ per milligram of the feed, or by dipping the fish in a solution containing said aquabirnavirus at a rate of 10⁵ TCID₅₀ to 10⁶ per millimeter of the solution.
